# FASCICULE DE BREVET EUROPEEN

(11) **EP 2 146 714 B1**
(45) Date de publication et mention de la délivrance du brevet: **22.06.2011**
(21) Numéro de dépôt: 08787963.1
(22) Date de dépôt: 16.04.2008
(51) Int. Cl.: A61K 31/444, A61P 25/16

(54) **UTILISATION DU 4-CYCLOPROPYLMETHOXY-N-(3,5-DICHLORO-1 -OXYDO- PYRIDIN-4-YL)-5-(METHOXY)PYRIDINE-2-CARBOXAMIDE POUR LE TRAITEMENT DES DESORDRES MOTEURS LIES A LA MALADIE DE PARKINSON**
VERWENDUNG VON 4-CYCLOPROPYLMETHOXY-N-(3,5-DICHLOR-1-OXIDOPYRIDIN-4-YL)-5-(METHOXY-)PYRIDIN-2-CARBOXAMID ZUR BEHANDLUNG MOTORISCHER STÖRUNGEN AUFGRUND DER PARKINSONSCHEN KRANKHEIT
USE OF 4-CYCLOPROPYLMETHOXY-<I>N</I>-(3,5-DICHLORO-1-OXIDOPYRIDIN-4-YL)-5-(METHOXY)PYRIDINE-2-CARBOXAMIDE FOR THE TREATMENT OF MOTOR DISORDERS RELATED TO PARKINSON'S DISEASE

(30) Priorité: 19.04.2007 FR 0702853
(43) Date de publication de la demande: 27.01.2010
(73) Titulaire: Sanofi-Aventis, 75013 Paris (FR)
(72) Inventeur: DELAY-GOYET, Philippe, F-75013 Paris (FR); DELGORGE, Claire, F-75013 Paris (FR); MENET, Christine, F-75013 Paris (FR); POUGHON, Gilles, F-75013 Paris (FR); RAVINET-TRILLOU, Christine, F-75013 Paris (FR)
(74) Mandataire: Gaslonde, Aude
(86) Numéro de dépôt international: PCT/FR2008/000534
(87) Numéro de publication internationale: WO 2008/145841

(56) Documents cités:
- WO-A-95/04045
- WO-A-2004/005258

## Description

La présente invention a pour objet l'utilisation du 4-cyclopropylméthoxy*-N-*(3,5-dichloro-1-oxydo-pyridin-4-yl)-5-(méthoxy)pyridine-2-carboxamide sous forme d'hydrate, de solvate, de base ou de sel d'addition à un acide, pour la préparation d'un médicament destiné au traitement des désordres moteurs liés à la maladie de Parkinson.

Le 4-cyclopropylméthoxy*-N-*(3,5-dichloro-l-oxydo-pyridin-4-yl)-5-(méthoxy)pyridine-2-carboxamide ou encore appelé *N*-(3,5-dichloro-1-oxydo-4-pyridinio)-4-cyclopropylméthoxy-5-méthoxypyridine-2-carboxamide, est connu pour entrer dans la composition de médicaments destinés au traitement de différentes pathologies dont notamment les inflammations articulaires, l'arthrite, l'arthrite rhumatoïde, ainsi que pour le traitement des désordres de la mémoire liés à la maladie d'Alzheimer. Ce composé, sous forme hémihydrate, est décrit par exemple dans le document WO95104045 (composé référencé FR).

Il existe un besoin de trouver des médicaments permettant de traiter en préventif les patients contre l'aggravation des désordres moteurs qui sont liés à la maladie de Parkinson. Des études chez l'animal ont montré qu'une voie possible est l'administration de composés pouvant inhiber les phosphodiestérases 4 (PDE 4), tels que par exemple le rolipram. Toutefois, des études cliniques ont montré que ce composé, ainsi que d'autres inhibiteurs de PDE 4, induit des effets émétisants qui ne permettent pas son utilisation en thérapie.

On a trouvé maintenant que le 4-cyclopropylméthoxy*-N-*(3,5-dichloro-1-oxydo-pyridin-4-yl)-5-(méthoxy)pyridine-2-carboxamide peut être utilisé dans le traitement des désordres moteurs liés à la maladie de Parkinson, tout en évitant les effets émétisants à des doses thérapeutiques acceptables.

Un premier objet de l'invention concerne donc l'utilisation du 4-cyclopropylméthoxy*-N-*(3,5-dichloro-1-oxydo-pyridin-4-yl)-5-(méthoxy)pyridine-2-carboxamide pour la préparation d'un médicament destiné au traitement des désordres moteurs liés à la maladie de Parkinson.

Selon un mode d'exécution de l'invention, l'utilisation du 4-cyclopropylméthoxy*-N-*(3,5-dichloro-1-oxydo-pyridin-4-yl)-5-(méthoxy)pyridine-2-carboxamide peut se faire sous forme de base ou de sel d'addition à un acide.

Les sels utilisables dans le cadre de l'invention peuvent être préparés avec des acides pharmaceutiquement acceptables, mais les sels d'autres acides utiles, par exemple, pour la purification ou l'isolement du 4-cyclopropylméthoxy*-N-*(3,5-dichloro-1-oxydo-pyridin-4-yl)-5-(méthoxy)pyridine-2-carboxamide, font également partie de l'invention.

L'utilisation du 4-cyclopropylméthoxy*-N-*(3,5-dichloro-1-oxydo-pyridin-4.-yl)-5-(méthoxy)pyridine-2-carboxamide selon l'invention peut se faire également sous forme d'hydrate ou de solvate. Par hydrate ou solvate, on entend l'association ou la combinaison de une ou plusieurs molécules de 4-cyclopropylméthoxy*-N-*(3,5-dichloro-1-oxydo-pyridin-4-yl)-5-(méthoxy)pyridine-2-carboxamide avec une ou plusieurs molécules d'eau ou de solvant.

Au sens de la présente invention, on entend par désordre moteur lié à la maladie de Parkinson les désordres suivants : bradykinésie, akinésie, rigidité, désordres et instabilité posturaux, perturbation de la démarche, tremblements, troubles de l'expression écrite et orale, dysphagie, troubles respiratoires, troubles vésico-sphinctériens.

Un second objet de l'invention concerne une composition pharmaceutique comprenant, à titre de principe actif, le 4-cyclopropylméthoxy*-N-*(3,5-dichloro-1-oxydo-pyridin-4-yl)-5-(méthoxy)pyridine-2-carboxamide, et un ou plusieurs excipients pharmaceutiquement acceptables pour l'utilisation telle que revendiquée.

La composition utilisée selon l'invention comprend une dose efficace du principe actif.

Par exemple, les doses journalières de principe actif utilisable selon l'invention sont de 0,001 à 10 mg/jour.

Selon la pratique habituelle, le dosage approprié à chaque patient est déterminé par le médecin selon le mode d'administration, l'âge, le poids et la réponse dudit patient.

Les doses dépendent de l'effet recherché, de la durée du traitement et de la voie d'administration utilisée.

II peut y avoir des cas particuliers où des dosages plus élevés ou plus faibles sont appropriés. De tels dosages ne sortent pas du cadre de l'invention.

Les excipients sont choisis selon la forme pharmaceutique et le mode d'administration souhaité, parmi les excipients habituels qui sont connus de l'homme du métier.

La composition peut être administrée par voie orale, parentérale ou rectale.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intra musculaire, intraveineuse ou intrathécale, les formes d'administration rectale et les implants. Pour l'application topique, on peut utiliser les principes actifs selon l'invention dans des crèmes, gels, pommades ou lotions.

Lorsqu'on prépare une composition sous forme de comprimé, on mélange le principe actif avec un ou plusieurs excipients pharmaceutiques, tels que la gélatine, l'amidon, le lactose, le stéarate de magnésium, le talc, la silice, la gomme arabique, le mannitol, la cellulose microcristalline, l'hypromellose ou analogues.

On peut enrober les comprimés de saccharose, d'un dérivé cellulosique ou d'autres matières adaptées à l'enrobage. Les comprimés peuvent être réalisés par différentes techniques, telles que la compression directe, la granulation sèche ou humide ou la fusion à chaud.

On peut également obtenir une composition pharmaceutique sous forme de gélule en mélangeant le principe actif avec un diluant et en versant le mélange obtenu dans des gélules molles ou dures.

Pour une administration parentérale, on utilise des suspensions aqueuses, des solutions salines isotoniques ou des solutions stériles et injectables qui contiennent des agents pharmacologiquement compatibles, par exemple le propylèneglycol ou le butylèneglycol.

A titre d'exemple, une forme unitaire d'administration de 4-cyclopropylméthoxy*-N-*(3,5-dichloro-1-oxydo-pyridin-4-yl)-5-(méthoxy)pyridine-2-carboxamide sous forme de comprimé comprend les ingrédients suivants :

| | |
|---|---|
| 4-cyclopropylméthoxy-*N*-(3,5-dichloro-1-oxydo-pyridin-4-yl)-5-(méthoxy)pyridine-2-carboxamide | 1 mg |
| Mannitol | 224 mg |
| Croscarmellose sodique | 5 mg |
| Amidon de maïs | 15 mg |
| Hydroxypropyl-méthylcellulose | 2 mg |
| Stéarate de magnésium | 3 mg |

Les effets du 4-cyclopropylméthoxy*-N-*(3,5-dichloro-1-oxydo-pyridin-4-yl)-5-(méthoxy)pyridine-2-carboxamide utilisé selon l'invention ont été évalués chez la souris à partir d'un modèle de perte de neurone dopaminergique. Exemple 1 : Protection dans un modèle de la maladie de Parkinson (perte de neurones

### dopaminergiques chez la souris intoxiquée au MPTP (1-Méthyl-4-phényl-1,2,3,6-tétrahydropyridine) :

Des souris C57BL6 reçoivent 4 injections de 20 mg/kg de MPTP par voie intra-péritonéale, séparées chacune de 2 heures. Le 4-cyclopropylméthoxy*-N-*(3,5-dichloro-1-oxydo-pyridin-4-yl)-5-(méthoxy)pyridine-2-carboxamide en solution dans le véhicule (méthylcellulose (MC) (0,6%) + Tween-80 (0,5%)) est administré par gavage entre la 2ème et la 3ème injection de MPTP et juste après la dernière injection de MPTP, puis 2 fois par jour pendant 17 jours aux doses totales journalières de 0,015 et 0,050 mg/kg. Vingt-quatre heures après le dernier traitement, le striatum est prélevé et les sites de capture de la dopamine sont quantifiés par une méthode de liaison du GBR12935 (1-[2-(diphényl-méthoxy)éthyl]-4-(3-phénylpropyl)-pipérazine).

Après injection de MPTP, la densité des sites de capture de la dopamine correspond seulement à 58% (p<0,01) de celle mesurée chez les animaux sains. Le traitement avec le 4-cydopropylméthoxy*-N-*(3,5-d ichloro-1-oxydo-pyridin-4-yl)-5-(méthoxy)pyridine-2-carboxamide a montré une capacité à protéger de la diminution induite par le MPTP : la densité des sites de capture de la dopamine atteint 82% et 85% du niveau observé chez les animaux sains respectivement aux doses de 0,015 et 0,050 mg/kg/j (p<0,01 en comparaison avec les animaux recevant seulement le MPTP).

### Exemple 2 : Evaluation des effets émétisants du 4-cyclopropylméthoxy-N-(3,5-dichloro-1-oxydo-pyridin-4-yl)-5-(méthoxy)pyridine-2-carboxamide.

Le pouvoir émétique du 4-cyclopropylméthoxy*-N-*(3,5-dichloro-1-oxydo-pyridin-4-yl)-5-(méthoxy)pyridine-2-carboxamide a été évalué chez le furet. Deux groupes de furets ont été utilisés, le premier recevant le véhicule (PEG200) et le second le 4-cyclopropylméthoxy*-N-*(3,5-dichloro-1-oxydo-pyridin-4-yl)-5-(méthoxy)pyridine-2-carboxamide en solution dans le véhicule (PEG 200), par gavage oral. Les animaux ont été observés continuellement pendant les 2 heures suivant l'administration puis une fois par heure jusqu'à 6 heures après l'administration. Les signes cliniques (en particulier hauts-le-coeur et vomissements) ont été notés.

Administré à 0,1 mg/kg, le 4-cyclopropylméthoxy*-N-*(3,5-dichloro-1-oxydo-pyridin-4-yl)-5-(méthoxy)pyridine-2-carboxamide n'induit aucun haut-le-coeur ni vomissement chez les 5 furets traités.

Ces résultats montrent que l'administration d'une dose thérapeutique de 4-cyclopropylméthoxy*-N-*(3,5-dichloro-1-oxydo-pyridin-4-yl)-5-(méthoxy)pyridine-2-carboxamide pour traiter des désordres moteurs liés à la maladie de Parkinson, n'entraîne pas d'effet émétique.

### Exemple 3: Evaluation des effets émétisants du (R)-(-)-rolipram (((4R)-4-[3-(Cyclopentyloxy)-4-methoxyphenyl]pyrrolidine-2-one).

Le pouvoir émétique du (*R*)-(-)-rolipram a été évalué chez le furet. Deux groupes de furets ont été utilisés, le premier recevant le véhicule (PEG200) et le second le 4-cyclopropylméthoxy*-N-*(3,5-dichloro-1-oxydo-pyridin-4-yl)-5-(méthoxy)pyridine-2-carboxamide en solution dans le véhicule (PEG 200), par gavage oral, aux doses de 0,05 mg/kg et de 0,1 mg/kg. Les animaux ont été observés continuellement pendant les 2 heures suivant l'administration puis une fois par heure jusqu'à 6 heures après l'administration. Les signes cliniques ont été notés.

Administré à 0,05 mg/kg et 0,1 mg/kg, le (*R*)-(-)-rolipram induit des vomissements chez les furets traités.

Les résultats de l'exemple 3 montre que l'administration d'une dose thérapeutique de (*R*)-(-)-Rolipram entraîne des effets émétiques.

Ainsi, le 4-cyclopropylméthoxy*-N-*(3,5-dichloro-1-oxydo-pyridin-4-yl)-5-(méthoxy)pyridine-2-carboxamide est utile dans la préparation d'un médicament pour le traitement des désordres moteurs liés à la maladie de Parkinson, tout en évitant d'éventuels effets émétisants.

## Revendications

1. Utilisation du 4-cyclopropylméthoxy-*N*-(3,5-dichloro-1-oxydo-pyridin-4-yl)-5-(méthoxy)pyridine-2-carboxamide, sous forme d'hydrate, de solvate, de base ou de sel d'addition à un acide, pour la préparation d'un médicament destiné au traitement des désordres moteurs liés à la maladie de Parkinson.

2. Utilisation selon la revendication 1, **caractérisée en ce que** le 4-cyclopropylméthoxy*-N-*(3,5-dichloro-1-oxydo-pyridin-4-yl)-5-(mèthoxy)pyridine-2-carboxamide est sous forme de base.

3. Utilisation selon l'une quelconque des revendications 1 à 2, **caractérisée en ce que** les désordres moteurs liés à la maladie de Parkinson sont bradykinésie, akinésie, rigidité, désordres et instabilité posturaux, perturbation de la démarche, tremblements, troubles de l'expression écrite et orale, dysphagie, troubles respiratoires, troubles vésico-sphinctériens.

4. Utilisation selon l'une quelconque des revendications 1 à 3, évitant les effets émétisants.

5. Utilisation selon la revendication 4, n'induisant ni haut-le-coeur ni vomissement.

## Claims

1. Use of 4-cyclopropylmethoxy*-N-*(3,5-dichloro-1-oxidopyridin-4-yl)-5-methoxypyridine-2-carboxamide, in the hydrate, solvate or base form or in the form of an additional salt with an acid, in the preparation of a medicament intended for the treatment of motor disorders related to Parkinson's disease.

2. Use according to Claim 1, **characterized in that** 4-cyclopropylmethoxy*-N-*(3,5-dichloro-1-oxido-pyridin-4-yl)-5-methoxypyridine-2-carboxamide is in the base form.

3. Use according to either one of Claims 1 and 2, **characterized in that** the motor disorders related to Parkinson's disease are bradykinesia, akinesia, stiffness, postural disorders and instability, gait disturbance, trembling, disorders of written and oral expression, dysphagia, respiratory disorders or vesical sphincter disorders.

4. Use according to any one of Claims 1 to 3, avoiding emetic effects.

5. Use according to Claim 4, inducing neither retching nor vomiting.

## Patentansprüche

1. Verwendung von 4-Cyclopropylmethoxy*-N-*(3,5-dichlor-1-oxidopyridin-4-yl)-5-(methoxy)pyridin-2-carboxamid in Form eines Hydrates, Solvates, einer Base oder eines Säureadditionssalzes zur Herstellung eines Arzneimittels für die Behandlung motorischer Störungen in Verbindung mit der Parkinson-Krankheit.

2. Verwendung nach Anspruch 1, **dadurch gekennzeichnet, dass** 4-Cyclopropylmethoxy*-N-*(3,5-dichlor-1-oxido-pyridin-4-yl)-5-(methoxy)pyridin-2-carboxamid in Form der Base vorliegt.

3. Verwendung nach einem der Ansprüche 1 bis 2, **dadurch gekennzeichnet, dass** es sich bei den motorischen Störungen in Verbindung mit der Parkinson-Krankheit um Bradykinesie, Akinesie, Steifheit, Störungen und Instabilitäten der Haltung, Gehstörung, Tremor, Störungen des schriftlichen und mündlichen Ausdrucks, Dysphagie, Atemstörungen, Vesiko-Sphinkter-Störungen handelt.

4. Verwendung nach einem der Ansprüche 1 bis 3, wobei Brechreiz erzeugende Wirkungen vermieden werden.

5. Verwendung nach Anspruch 4, wobei weder Übelkeit noch Erbrechen hervorgerufen werden.
